Europäisches Patentamt

European Patent Office

Office européen des brevets

(19)

(11)     EP 1 167 337 B1

(12)        **FASCICULE DE BREVET EUROPEEN**

(45)  Date de publication et mention
de la délivrance du brevet:
**16.04.2003   Bulletin 2003/16**

(51)  Int Cl.⁷: **C07C 67/08**, C07C 69/30,
C07C 69/54

(21)  Numéro de dépôt: **01401444.3**

(22)  Date de dépôt: **05.06.2001**

(54)  **Préparation d'esters carboxyliques catalysée par un acide sulfonique**

Durch eine Sulfonsäure katalysierte Herstellung von Carbonsäureester

Preparation of esters of carboxylic acids catalysed by a sulfonic acid

(84)  Etats contractants désignés:
**AT BE CH CY DE DK ES FI FR GB GR IE IT LI LU
MC NL PT SE TR**

(30)  Priorité: **19.06.2000   FR 0007784**

(43)  Date de publication de la demande:
**02.01.2002   Bulletin 2002/01**

(73)  Titulaire: **Atofina
92091 Paris La Défense Cedex (FR)**

(72)  Inventeur: **Gancet, Christian
64140 Lons (FR)**

(74)  Mandataire: **Granet, Pierre
Atofina,
Département Propriété Industrielle,
4-8, cours Michelet,
La Défense 10
92091 Paris La Défense Cedex (FR)**

(56)  Documents cités:
**EP-A- 0 775 687          DE-A- 4 017 903**

EP 1 167 337 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention concerne le domaine des esters carboxyliques et a plus particulièrement pour objet leur synthèse par réaction directe d'un acide carboxylique et d'un alcool.

Cette réaction est généralement catalysée par un acide qui peut être l'acide sulfurique ou une résine acide, mais très fréquemment un acide sulfonique $R-SO_3H$ tel que l'acide méthanesulfonique car les acides sulfoniques permettent, entre autres avantages, d'obtenir des esters carboxyliques de meilleure qualité. Cependant, les acides sulfoniques et en particulier l'acide méthanesulfonique présentent l'inconvénient de former durant la réaction une faible quantité d'esters sulfoniques de l'alcool qui entraînent la présence de soufre résiduel dans l'ester carboxylique désiré et dont l'hydrolyse génère la formation d'acidité libre néfaste à la qualité du produit.

Dans le cas des esters carboxyliques légers distillables sans dégradation, le problème peut être résolu en utilisant un acide sulfonique lourd dont l'ester formé peut être séparé de l'ester carboxylique par distillation. Cependant, cette solution ne convient pas dans le cas des esters carboxyliques dégradables; le produit réactionnel doit alors être traité séparément sur une résine pour éliminer l'ester sulfonique formé.

On sait qu'une réaction de quaternisation irréversible intervient entre une amine tertiaire $NR^1R^2R^3$ et un composé donneur d'alkyle tel qu'un ester sulfonique $R-SO_3$-Alkyl selon le schéma suivant:

$$R-SO_3\text{-Alkyl} + NR^1R^2R^3 \rightarrow R^1R^2R^3\text{Alkyl}N^+ \; R-SO_3^-$$

Cette réaction a même été utilisée pour réaliser des post-traitements d'esters de façon à en éliminer certains esters sulfoniques présents. Cependant, ceci impose une étape supplémentaire dans le procédé de fabrication des esters et n'est donc pas facile à mettre en oeuvre.

Il a maintenant été trouvé que cette réaction de quaternisation peut se produire pendant la réaction de synthèse des esters carboxyliques elle-même. Non seulement, la réaction de quaternisation est possible et permet d'éliminer les esters sulfoniques au fur et à mesure de leur formation, mais en outre la présence de l'amine tertiaire ne modifie en rien les performances du catalyseur acide sulfonique.

L'invention a donc pour objet un procédé pour la préparation d'un ester carboxylique par réaction directe d'un acide carboxylique et d'un alcool catalysée par un acide sulfonique $R-SO_3H$, caractérisé en ce que l'on opère en présence d'un solide comportant des fonctions amine tertiaire sous forme libre.

Comme solides comportant des fonctions amine tertiaire, on peut utiliser conformément à la présente invention des polymères ou copolymères organiques à fonctions amine tertiaire, bien connus dans l'art comme échangeurs d'anions. Comme tels, on peut employer plus particulièrement des résines à base de polystyrène réticulées notamment avec du divinylbenzène, des résines acryliques ou phénylacryliques, des résines acryliques réticulées avec du divinylbenzène, ou des résines du type phénol-formaldéhyde. Ces résines portent des groupes fonctionnels amine tertiaire fixés par différentes techniques connues en soi, généralement après formation des polymères ou copolymères. On peut mentionner également les poly-vinylpyridines obtenues par polymérisation de la 4-vinylpyridine. De telles résines échangeuses d'anions se trouvent dans le commerce sous différentes dénominations telles que, par exemple, Amberlite, Duolite, IRA et Reillex.

La quantité de solide à mettre en oeuvre peut varier dans de larges limites. Elle est généralement comprise entre 0,01 et 20 % par rapport au poids du mélange réactionnel (acide + alcool + solvant éventuel), de préférence entre 0,1 et 5 %.

Le procédé selon l'invention peut s'appliquer à la préparation de tout ester carboxylique dont la synthèse directe à partir de l'acide carboxylique et de l'alcool correspondants est catalysée par un acide sulfonique $R-SO_3H$. Il s'applique notamment à la synthèse des esters dérivés des acides carboxyliques tels que les acides acryliques ou méthacryliques ou les acides gras comme l'acide heptanoïque ou l'acide stéarique, et des alcools mono, di ou polyfonctionnels tels que l'isopropanol, le 2-éthylhexanol, le di-isopropylène-glycol, le di-isobutylène-glycol, les polyéthylène-glycols ou le triméthylol-propane.

Comme acides sulfoniques $R-SO_3H$, on peut mentionner plus particulièrement ceux dans lesquels R est un radical aliphatique ou aromatique, tels que les acides $CH_3(CH_2)_nSO_3H$ avec n allant de 0 à 3 et l'acide p-toluènesulfonique. L'acide sulfonique préféré est l'acide méthanesulfonique.

La réaction d'estérification proprement dite est réalisée dans les conditions opératoires habituelles, c'est-à-dire:

- rapport molaire acide carboxylique/alcool allant de 1 à 10, de préférence compris entre 1 et 5,
- température comprise entre 50 et 200°C, de préférence entre 80 et 150°C,
- quantité d'acide sulfonique $R-SO_3H$ comprise entre 0,1 et 5 % par rapport au poids d'acide carboxylique, de préférence entre 0,5 et 2 %.

On peut opérer en l'absence de solvant ou en mélange avec un solvant tel que le toluène ou l'heptane. La quantité de solvant peut varier dans de larges limites, mais elle est généralement comprise entre 5 et 95 % par rapport au poids du mélange réactionnel (acide + alcool + solvant), de préférence entre 5 et 50 %.

La réaction d'estérification peut être réalisée en discontinu, semi-continu ou continu.

En fin de réaction, le solide peut être séparé par simple filtration ou tout autre moyen équivalent.

Les exemples suivants qui illustrent l'invention sans la limiter, concernent l'estérification du triméthylolpropane (TMP) par l'acide heptanoïque.

**EXEMPLE 1 Comparatif**

Dans un ballon placé dans un bain chauffant et équipé d'un réfrigérant et d'un Dean Stark, on a introduit 60 g (0,46 mole) d'acide heptanoïque, 20 g (0,149 mole) de triméthylolpropane et 100 ml de toluène, puis on a mis en marche le bain chauffant et fixé à 150°C la température de consigne.

Lorsque le reflux du toluène a commencé dans le Dean Stark (à environ 120°C), on a injecté 1,2 g d'acide méthanesulfonique anhydre et, après 90 minutes, on a enlevé le bain chauffant et laissé refroidir le mélange pendant environ une heure. On a ensuite neutralisé avec 2 fois un volume de solution aqueuse de carbonate de sodium à 100 g/l correspondant à 10 % du volume réactionnel, puis lavé 4 fois avec un volume d'eau correspondant à 10 % du volume réactionnel, jusqu'à neutralisation de la phase aqueuse.

Après séparation des phases, on a évaporé le toluène à 65°C sous un vide d'environ 25 mbar, puis pesé le produit final et procédé à son analyse par chromatographie gazeuse. Le produit contenait 91,7 % en poids de triheptanoate de triméthylolpropane (THTMP) et 0,47 % en poids d'ester sulfonique.

**EXEMPLE 2 Comparatif**

On a répété l'exemple 1, mais en opérant avec un défaut d'acide heptanoïque (48 g) et un excès (10 g) d'acide méthanesulfonique anhydre.

Le produit final contenait 51,4 % en poids de triheptanoate de triméthylolpropane (THTMP) et 5,6 % en poids d'ester sulfonique.

L'analyse par chromatographie gazeuse et par spectrométrie de masse a montré que l'ester sulfonique principalement obtenu est l'ester diheptanoïque et méthanesulfonique du triméthylolpropane.

**EXEMPLE 3**

On a répété l'exemple 1, mais en ajoutant 0,24 g (2,3 mmoles) de poly-vinylpyridine (PVP Reillex 402), soit 20 % en poids par rapport à l'acide méthanesulfonique.

Le produit final contenait 92,2 % en poids de triheptanoate de triméthylolpropane (THTMP) et seulement 0,11 % en poids d'ester sulfonique.

**EXEMPLE 4 à 6**

On a répété l'exemple 2, mais en ajoutant des quantités variables de polyvinylpyridine (PVP Reillex 402), à savoir 26 %, 58 % et 100 % en poids par rapport à l'acide méthanesulfonique.

Les résultas obtenus sont rassemblés dans le tableau suivant.

| Exemple | 2 | 4 | 5 | 6 |
|---|---|---|---|---|
| PVP (g) | 0 | 2,6 | 5,8 | 10 |
| THTMP (%) | 51,4 | 55,6 | 52,4 | 58,9 |
| Ester sulfonique (%) | 5,6 | 2,6 | 0,22 | 0,1 |

**EXEMPLE 7 à 10**

Les conditions d'estérification sont celles de l'exemple 1. Les résines utilisées sont respectivement la polyvinypyridine précédente à titre de comparaison, l'Amberlite IRA 96 de Rohm & Haas (squelette styrène-divinylbenzène), l'Amberlite IRA 92 de Rohm & Haas (squelette polystyrène macroporeux), et la Duolite A561 de Rohm & Haas (squelette phénol-formaldéhyde réticulé).

Les résultas obtenus sont rassemblés dans le tableau suivant :

| Exemple | Résine | Masse de résine (g) | Soufre résiduel dans l'ester final (mg/l) |
|---------|--------|---------------------|-------------------------------------------|
| 1 | néant | 0 | 250 |
| 7 | Reillex 402 | 0,46 | 112 |
| 8 | IRA 96 | 0,69 | 98 |
| 9 | IRA 92 | 0,79 | 98 |
| 10 | Duolite A561 | 0,42 | 130 |

Les résines des exemples 8, 9 et 10, livrées humides et sous forme amine libre, sont lavées au méthanol et séchées avant utilisation.

**Revendications**

1. Procédé pour la préparation d'un ester carboxylique par réaction directe d'un acide carboxylique et d'un alcool catalysée par un acide sulfonique R-SO$_3$H, **caractérisé en ce que** l'on opère en présence d'un solide comportant des fonctions amine tertiaire sous forme libre.

2. Procédé selon la revendication 1 dans lequel le solide est une polyvinylpyridine.

3. Procédé selon la revendication 1 dans lequel le solide est une résine de type styrène-divinylbenzène, polystyrène ou phénol-formaldéhyde.

4. Procédé selon l'une des revendications 1 à 3 dans lequel la quantité de solide mise en oeuvre est comprise entre 0,01 et 20 % par rapport au poids du mélange réactionnel, de préférence entre 0,1 et 5 %.

5. Procédé selon l'une des revendications 1 à 4 dans lequel l'acide sulfonique est l'acide méthanesulfonique.

6. Procédé selon l'une des revendications 1 à 5 dans lequel l'acide carboxylique est l'acide acrylique, l'acide méthacrylique, l'acide heptanoïque ou l'acide stéarique.

7. Procédé selon l'une des revendications 1 à 6 dans lequel l'alcool est l'isopropanol, le 2-éthylhexanol, le di-isopropylène-glycol, le di-isobutylène-glycol, les polyéthylène-glycols ou le triméthylol-propane.

**Claims**

1. Process for the preparation of a carboxylic ester by direct reaction of a carboxylic acid and an alcohol catalysed by a sulphonic acid R-SO$_3$H, **characterized in that** the procedure is carried out in the presence of a solid comprising tertiary amine functions in free form.

2. Process according to Claim 1, in which the solid is a polyvinylpyridine.

3. Process according to Claim 1, in which the solid is a resin of the styrene-divinylbenzene, polystyrene or phenol-formaldehyde type.

4. Process according to one of Claims 1 to 3, in which the quantity of solid used is between 0.01 and 20% relative to the weight of the reaction mixture, preferably between 0.1 and 5%.

5. Process according to one of Claims 1 to 4, in which the sulphonic acid is methanesulphonic acid.

6. Process according to one of Claims 1 to 5, in which the carboxylic acid is acrylic acid, methacrylic acid, heptanoic acid or stearic acid.

7. Process according to one of Claims 1 to 6, in which the alcohol is isopropanol, 2-ethylhexanol, diisopropylene glycol, diisobutylene glycol, polyethylene glycols or trimethylolpropane.

**Patentansprüche**

1. Verfahren zur Herstellung eines Carbonsäureesters mittels direkter, durch eine Sulfonsäure R-SO$_3$H katalysierter Reaktion einer Carbonsäure mit einem Alkohol, **dadurch gekennzeichnet, daß** man in Gegenwart eines Feststoffs mit tertiären Aminfunktionen in freier Form verfährt.

2. Verfahren nach Anspruch 1, wobei der Feststoff ein Polyvinylpyridin ist.

3. Verfahren nach Anspruch 1, wobei der Feststoff ein Harz vom Typ Styrol/Divinylbenzol, Polystyrol oder Phenol/ Formaldehyd ist.

4. Verfahren nach einem der Ansprüche 1 bis 3, wobei die eingesetzte Feststoffmenge zwischen 0,01 und 20 %, vorzugsweise zwischen 0, 1 und 5 %, bezogen auf das Gewicht der Reaktionsmischung, liegt.

5. Verfahren nach einem Ansprüche 1 bis 4, wobei die Sulfonsäure Methansulfonsäure ist.

6. Verfahren nach einem der Ansprüche 1 bis 5, wobei die Carbonsäure Acrylsäure, Methacrylsäure, Heptansäure oder Stearinsäure ist.

7. Verfahren nach einem der Ansprüche 1 bis 6, wobei der Alkohol Isopropanol, 2-Ethylhexanol, Diisopropylenglykol, Diisobutylenglykol, Polyethylenglykole oder Trimethylolpropan ist.